(19) Europäisches Patentamt
European Patent Office
Office européen des brevets

(11) **EP 4 364 669 A1**

(12) **EUROPEAN PATENT APPLICATION**

(43) Date of publication:
**08.05.2024 Bulletin 2024/19**

(21) Application number: **22205202.9**

(22) Date of filing: **03.11.2022**

(51) International Patent Classification (IPC):
**A61B 7/00** (2006.01)    **A61B 5/08** (2006.01)

(52) Cooperative Patent Classification (CPC):
**A61B 7/003; A61B 5/0803; A61B 5/6823;**
**A61B 5/7257; A61B 5/7264; A61B 5/7282;**
A61B 2562/0204

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(71) Applicants:
• **Université de Genève - UNIGE**
  **1211 Genève (CH)**
• **Hopitaux Universitaires de Genève**
  **1205 Genève (CH)**

(72) Inventors:
• **Heitmann, Julien**
  **1205 Genève (CH)**
• **Glangetas, Alban**
  **1205 Genève (CH)**
• **Gervaix, Alain**
  **1205 Genève (CH)**

(74) Representative: **KATZAROV S.A.**
  **Geneva Business Center**
  **12 Avenue des Morgines**
  **1213 Petit-Lancy (CH)**

(54)    **DETECTION A RESPIRATORY DISEASE BASED ON CHEST SOUNDS**

(57)    The present relates to a method for detecting a disease in a patient comprising
providing chest recordings of the patient,
processing the chest recordings with a computational tool,
detecting the presence or the absence of said disease depending on the output of the computational tool,
the chests recordings are recorded from a plurality of distinct acquisition sites distributed on the patient's chests,
the processing of the chest recordings comprises
i) applying a first computation tool on the chest recordings of each of acquisition site to compute a first score for each recording of each of acquisition site;
ii) aggregating all the first scores computed for the patient by applying a second computational tool to compute a global score for the patient for said disease;
iii) determining the presence or the absence of the disease based on the global score.

Figure 2

**Description**

**Technical Field**

The present invention relates to a method, preferably a computer implemented method, for detecting a disease in a patient.

**Background of the art**

Respiratory diseases are a diverse range of pathologies affecting the upper and lower airways (pharynx, trachea, bronchi, bronchioles), lung parenchyma (alveoli) and its covering (pleura). The restriction of air flow in the variously sized passageways creates distinct patterns of sound that are detectable with stethoscopes as abnormal, "adventitious" sounds such as wheezing, rhonchi and crackles that indicate airflow resistance or the audible movement of pathological secretions. While there are some etiological associations with these sounds, the causal nuances are difficult to interpret by humans, due to the diversity of differential diagnoses and the non-specific, unstandardized nomenclature used to describe auscultation.

Indeed, despite two centuries of experience with conventional stethoscopes, during which time it has inarguably become one of the most ubiquitously used clinical tools, several studies have shown that the clinical interpretation of chest sounds, in particular lung sounds, is highly subjective and varies widely depending on the level of experience and specialty of the caregiver.

Various approaches have been reported to overcome the above-mentioned limitations. Recent advances in audio signal processing have shown that trained computational models, for instance deep learning, have potential to discriminate audio patterns more objectively, and out-perform human perception. Several studies have sought to automate the interpretation of digital lung auscultations (DLA), with several more recent ones using deep learning models. However, most studies aim to automate the detection of the adventitious sounds that were annotated by humans. Thus, integrating the limitations of human perception into the prediction. Further, as these pathological sounds do not have specific diagnostic/prognostic associations, the clinical relevance of these approaches is limited.

Chest auscultation is a ubiquitous clinical exam in the diagnosis of chest disease, in particular lung or respiratory disease, and its interpretation can influence care. Diagnostic uncertainty can thus contribute to why respiratory diseases are among the most widely misdiagnosed. Improvements in the interpretation this exam's results would not only improve patient care, but could have a major impact on antibiotic stewardship. Therefore, there is a need to provide an improved method for the detection of diseases based on chest auscultation.

**Summary of the invention**

The above problems are solved by the device and the method according to present invention.

The invention concerns a method, preferably a computer implemented method, for detecting a disease in a patient, the method comprising

providing chest recordings recorded during chest auscultation of the patient,

processing the chest recordings with a computational tool configured for recognizing audible signature corresponding to the disease,

detecting the presence or the absence of said disease depending on the output of the computational tool,

the method being characterized in that

the chest recordings are recorded from a plurality of distinct acquisition sites distributed on the patient's chest, each site corresponding to one recording;

and in that the processing of the chest recordings further comprises

i) applying a first computation tool on the chest recordings of each of acquisition site to compute a first score for each recording of each of acquisition site;

ii) aggregating all the first scores computed for the patient by applying a second computational tool to compute a global score for the patient for said disease;

iii) determining the presence or the absence of the disease based on the global score.

In another aspect, the invention concerns a computer program comprising instructions which, when the program is executed by a computer, cause the computer to carry out the method according to the present invention.

In another aspect, the invention relates to an apparatus, preferably a computer device, comprising means for carrying out the method of according to the present invention.

**Description of the invention**

The invention concerns a method, preferably a computer implemented method, for detecting a disease in a patient, the method comprising

providing chest recordings recorded during chest auscultation of the patient,

processing the chest recordings with a computational tool configured for recognizing audible signature corresponding to the disease,

detecting the presence or the absence of said disease depending on the output of the computational tool,

the method being characterized in that

the chest recordings are recorded from a plurality of distinct acquisition sites distributed on the patient's chest, each site corresponding to one recording;

and in that the processing of the chest recordings further comprises

i) applying a first computation tool on the chest recordings of each of acquisition site to compute a first score for each recording of each of acquisition site;

ii) aggregating all the first scores computed for the patient by applying a second computational tool to compute a global score for the patient for said disease;

iii) determining the presence or the absence of the disease based on the global score.

In the present invention, the chest recordings are recorded from a plurality of distinct acquisition sites distributed on the patient's chest, each site corresponding to one recording. Subsequently, the results of each site are processed by a computation tool to calculate a detection score (namely a global score) of the disease at the patient level. Having a single value for one patient, i.e. the global score, rather than multiple values (one for each acquisition site) facilitates the interpretation of the detection. This also minimizes or attenuates the negative impact of bad recordings for instance for one acquisition site (e.g. containing abnormal background noise).

In the present invention, chest (or thorax) recordings comprise sounds that can be recorded by auscultation a patient chest. Chest recordings comprise various body sounds of the patient such as (non exhaustive list) lung sounds, respiratory tract sounds, heart sounds.

Preferably, chest recordings are filtered to select the sound(s) relevant to the disease. For instance, chest recordings are filtered to select pulmonary sounds, preferably chest recordings are filtered to select pulmonary sounds, and preferably respiratory tract sounds, when the invention is used to detect respiratory diseases, preferably pulmonary disease. For instance, chest recordings are filtered to select pulmonary sounds and heart sounds when the invention is used to detect congenital or acquired heart diseases such as murmur.

Preferably, the first computation tool comprises at least one computation model trained for a specific disease and one model trained for identifying healthy patient so that the first computation tool provides a first score for each computation model for each site to compute the global score for the specific disease for the patient. This allows each disease-model to identify the audible signature (i.e. sounds) unique to its disease. This has better prospects than training a single multi-class model predicting the scores of all diseases.

Preferably, the first computation tool comprises several computation models each trained for a specific disease and one model trained for identifying healthy patient so that the first computation tool provides a first score for each computation model for each disease for each acquisition sites, to compute the global score for each specific disease for the patient. This integrates the information from all disease models to make better informed predictions on the probability of each disease.

Preferably, the computation model of the first computation tool comprises at least an audio classifier, preferably an CNN architecture comprising a plurality of convolutional blocks. CNNs have revolutionized the world of computer vision and have proven to be powerful computation tool in this area. Audio classification can also benefit from the advantages of CNN when the audio file is converted into a corresponding two dimensions image.

Preferably, the processing of the chest recordings further comprises a pre processing step comprising

a high pass filter to filter sounds of the chest recording below a cut-off frequency preferably of about 100 Hz, more preferably of about 125 Hz, more preferably of about 150 Hz,

and/or, preferably and

a low pass filter to filter sounds of the chest recording above a cut-off frequency preferably of about 1000 Hz, more preferably of about 900 Hz, more preferably of about 800 Hz. Advantageously, the filter removes the low-frequency sounds of the heart, and the high noise frequencies.

Preferably, each chest recording comprises an audio signal, the processing of the chest recordings further comprises
converting each audio signal into a two dimensions image preferably a spectrogram, preferably a log-mel spectrogram for instance by applying Discrete Fourier transform on each audio signal. This allows the further use of powerful neural networks specialized in image inputs like convolutional neural networks (CNN).

Preferably, the computation of the first score for each acquisition site comprises calculating segment-wise outputs for each site

aggregating the segment-wise outputs for each site into a single clip wise output as first score for each acquisition site

using said clip wise first score as input for the second computation tool.

These outputs, preferably the segment-wise output, indicate which parts of the recording are important for the global score.
Preferably, the computation model of the second computation tool comprises a logistic regression configured for aggregating all the first score of each acquisition site into a global score for the patient. The fitted coefficients of the logistic regression provide information on the relative weight of each acquisition site (i.e. position) for the global score.
Preferably, the determination step comprises

attributing a threshold score for said disease and

comparing the global score of the patient to the threshold score for said disease and

determining that the patient is positive for said disease if the global score of the patient is above the threshold score and that the patient is negative for said disease if the global score of the patient is below the threshold score.

Preferably, the threshold value has a default value, for instance 0.5 is used by default. If increased sensitivity or specificity is desired, the threshold value can be adapted accordingly by tuning the default value.
Preferably, the plurality of distinct acquisition sites is chosen among the list comprising sites in the right chest, sites in the left chest, in the superior part of the chest, sites in the inferior part of the chest, site in the anterior part of the chest, site in the posterior part of the chest,
preferably at least one site in the superior part of each chest, at least one site in the inferior part of each chest, at least one site in the anterior part of each chest, at least one site in the posterior part of each chest.
Preferably, the plurality of distinct acquisition sites is chosen among the list comprising sites in the right lung, sites in the left lung, in the superior part of the lungs, sites in the inferior part of the lungs, site in the anterior part of the lungs, site in the posterior part of the lungs,
preferably at least one site in the superior part of each lung, at least one site in the inferior part of each lung, at least one site in the anterior part of each lung, at least one site in the posterior part of each lung.
In a preferred embodiment, the plurality of distinct acquisition sites comprises at least 4 acquisitions sites, preferably between about 2 and 20 sites, preferably between 2 to 10, more preferably 4, more preferably 8.
Preferably, the duration of each recording is at least 3 seconds, preferably between about 3 seconds and 1 minute, preferably at least about 5 seconds, preferably between about 5 seconds and 35 seconds.
Preferably, the respiratory disease is chosen among wheezing disorders. Preferably, the wheezing disorder is chosen among asthma and bronchitis.
Preferably, the respiratory disease is chosen among pneumonia, asthma, bronchitis and bronchiolitis.

Preferably, the respiratory disease is chosen among pneumonia, asthma, bronchitis, bronchiolitis, asthma and bronchitis being pooled together as one disease.

More preferably, the respiratory diseases are pneumonia, asthma, bronchitis and bronchiolitis.

Preferably, the respiratory disease is pneumonia. Preferably, the respiratory disease is asthma. Preferably, the respiratory disease is bronchitis. Preferably, the respiratory disease is bronchiolitis.

Preferably, the disease is chosen among the respiratory disease, in particular pulmonary disease or bronchitis. Preferably, the respiratory disease is chosen among the list comprising Chronic obstructive pulmonary disease COPD, tuberculosis, COVID-19. Preferably, the respiratory disease is Chronic obstructive pulmonary disease COPD. Preferably, the respiratory disease is tuberculosis. Preferably, the respiratory disease is COVID-19.

Preferably, the disease is chosen among heart disease. For instance, the present invention allows to detect heart murmur in a patient. preferably the heart disease is chosen among the list comprising (non exhaustive list) congenital heart disease and acquired heart disease.

As used herein, the word "means" (singular or plural) preceded or followed by a function can be replaced by the word "unit" or " module". For instance "computation means" can be replaced by "computation unit" or "computation module".

The embodiments describe for the method also apply to the apparatus and computer program according to the present invention mutatis mutandis.

## Brief description of the drawings

Further particular advantages and features of the invention will become more apparent from the following non-limitative description of at least one embodiment of the invention which will refer to the accompanying drawings, wherein

- Figure 1 illustrates the data set partition strategy;
- Figure 2 represents an overview of the DeepBreath classification model;
- Figure 3 illustrates the feature construction for multi-class classification;
- Figure 4 represents the performance of the binary DeepBreath models on variable lengths and anatomical combinations of auscultation audio recordings.

## Detailed description of the invention

The present detailed description is intended to illustrate the invention in a non-limitative manner since any feature of an embodiment may be combined with any other feature of a different embodiment in an advantageous manner.

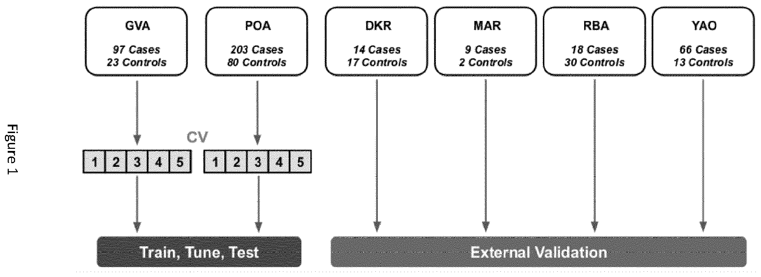

An example of the application of the present invention is explained below and on figures 1 to 4 with tables 1 and 2, but the invention is not limited to the present example.

In the present example, the respiratory diseases to be detected are (i) pneumonia, (ii) wheezing disorders, or (iii) bronchiolitis.

In the present invention, the chest recordings are pulmonary recordings, respiratory tract recordings and heart recordings.

The computation tool, namely *DeepBreath* in the present example, comprises a convolutional neural network as first computation tool followed by a logistic regression as second computation tool aggregating estimates on recordings from eight thoracic sites to a single prediction at the patient level.

Participants and cohort description : A total of 572 patients were recruited from two observational cohort studies. Of these, 71% (n=407/572) were clinically diagnosed cases with one of three diagnostic labels: (i) pneumonia, (ii) wheezing disorders, or (iii) bronchiolitis. The remaining 29% (n=165/572) were age- and sex-matched controls with no respiratory symptoms, who were consulting the same emergency unit for other complaints. All diagnoses are validated by two medical doctors. Pneumonia is diagnosed radiologically where possible or by the presence of audible crackles and/or febrile respiratory distress. Bronchiolitis and wheezing disorders (comprising asthma and obstructive bronchitis) are diagnosed clinically. A detailed breakdown of numbers stratified by geographic site and diagnostic label are provided in Table 1 below. Both studies are approved by the Research Ethics Committee of Geneva and local research ethics boards in each participating country. All patient's caregivers provided informed consent.

Pneumoscope. This is a multi-centre observational cohort study, comprising 529 pediatric outpatients under 16 years of age with suspected lower respiratory tract infection. A total of 174 age/sex-matched patients without respiratory symptoms served as controls. Patients with known chronic underlying respiratory disease (fibrosis etc.) or heart failure were excluded. All patients and controls were recruited between 2016 and 2020 at the outpatient departments of six hospitals across five countries (120 in Geneva, 102 Switzerland; 283 in Porto Alegre, Brazil; 31 in Dakar, Senegal; 79 in Yaounde, 103 Cameroon; and finally 59 from Rabat and Marrakesh in Morocco).

Asthmoscope. This is a single-centre observational cohort study comprising 43 pediatric outpatients aged between 2 and 16 years old presenting with wheezing disorders (asthma and/or obstructive bronchitis). Patients were recruited at the pediatric emergency department of Geneva University Hospitals (HUG) between 2019 and 2020.

Table 1

| | Control | Pneumonia | Bronchiolitis | Wheeze † | Total n |
|---|---|---|---|---|---|
| GVA | 23 | 27 | 4 | 66 | 120 |
| POA | 80 | 38 | 91 | 21 | Δ 283 |
| DKR | 17 | 5 | 1 | 8 | 31 |
| MAR / RBA | 32 | 0 | 8 | 19 | 59 |
| YAO | 13 | 44 | 16 | 6 | 79 |
| Total n | 165 | 114 | 120 | 120 | 572 |
| % | 29 | 20 | 21 | 21 | 100 |

Number of patients used to train the models in this study stratified by diagnosis and collection site.† Wheezing Disorder comprises obstructive bronchitis (30%) and asthma (70%); Δ In POA, 53 cases had no differentiation between bronchiolitis and wheezing disorder and are not listed in a specific column; GVA: Geneva, Switzerland; POA: Porto Alegre, Brazil; DKR: Dakar, Senegal; MAR:Marrakesh, Morocco; RBA: Rabat, Morocco; YAO: Yaounde, Cameroon.

Dataset - Acquisition. A series of digital lung auscultation (DLA) audios were acquired from each of the 572 recruited patients across eight anatomic sites (one in each quadrant of the anterior and posterior thorax). DLAs had an average duration of 28.4 seconds (range 1.9-30). Only 2.8% of patients (n=16/572) did not have all eight recordings present. Collectively, 4552 audio recordings covered 35.9 hours of breath sounds. All recordings were acquired on presentation, prior to any medical intervention (e.g. bronchodilators or supplemental oxygen). DLAs were recorded in WAVE (.wav) format with a Littmann 3200 electronic stethoscope (3M Health Care, St. Paul, USA) using the Littmann StethAssist proprietary software v.1.3 using the Bell Filter option. The stethoscope has a sampling rate of 4,000 Hz and a bit depth of 16 bits.

Dataset - Diagnostic labels. Recordings have one of four clinical diagnostic categories: control (healthy) patient, pneumonia, wheezing disorders (asthma and bronchitis pooled together) and bronchiolitis. The diagnosis was made by an experienced pediatrician after auscultation and was based on all available clinical and paraclinical information, such as chest X-ray when clinically indicated. For the purposes of this study, obstructive bronchitis and asthma are grouped under the label "wheezing disorder" due to their similar audible wheezing sound profile and treatment requirements (bronchodilators). Rather than having a single model with four output classes (listed above), we adopted a "one-versus-rest" approach where four separate binary classification models discriminate between samples of one class and samples of the remaining three classes.

The combination of the outputs of the resulting ensemble of four models yields a multi-class output. Such an approach allows for separating the learned features per class, which in turn improves the interpretability of the overall model. Moreover, such a composite model allows to more easily implement a sampling strategy that compensates for the imbalanced training sample size per class. For a fair comparison, an identical model architecture was used for all the diagnoses in this study. For a broader classification of pathological breath sounds, predictions from the three pathological classes are grouped into a composite "pathological" label.

Breath cycle annotations. Recordings from the Geneva site of the the Pneumoscope study (cases and controls) were annotated by medical doctors to provide time-stamped inspiration and expiration phases of the breath cycle. These will serve as a reference for the interpretation methods described below.

Data partitioning (train:tune:test split and external validation). Considering potential biases in the data collection process, and a model's tendency to overfit in the presence of site-specific background noise, it is particularly important to ensure balanced representation in data splits and even more critical to explicitly report results on an external validation set from an independent clinical setting. As the Geneva (GVA) and Porto Alegre (POA) recordings were the most abundant and diverse in terms of label representation, they were thus used for training, internal validation (i.e. tuning) and testing. To ensure that performance was not dependent on fortuitous data partitioning, 5-fold stratified cross validation was performed to obtain a distribution of performance estimates which are then reported as a mean with 95% confidence interval (95CI). The random fold compositions are restricted to maintain class balance (i.e. preserving the percentage of samples for each class). The other centres of Dakar (DKR), Marrakesh (MAR), Rabat (RBA) and Yaoundé (YAO) were used for external validation, i.e. the trained model has never seen recordings coming from these centres, and the recordings have not been used for hyper-parameter selection. The partitioning strategy is illustrated in Figure 1.

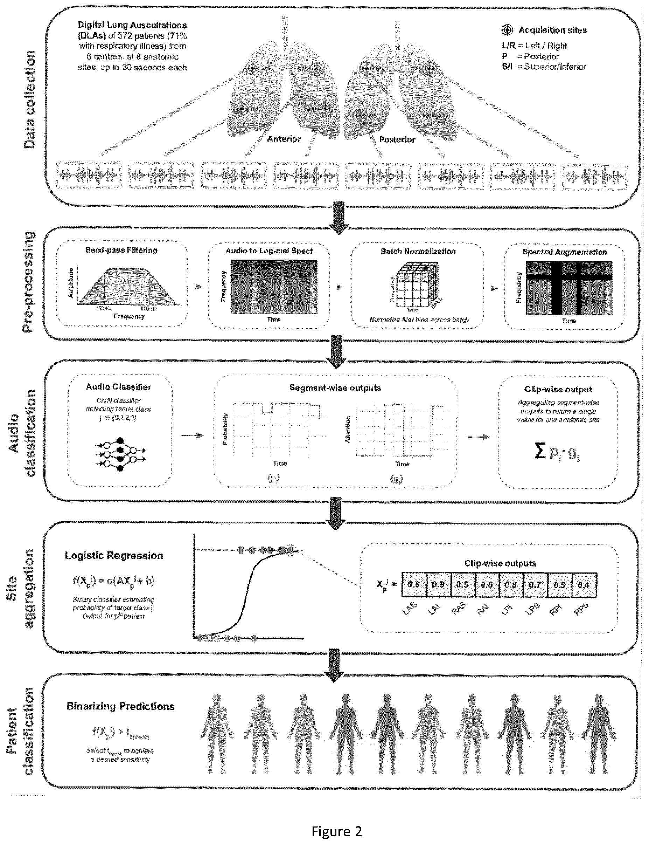

DeepBreath: DeepBreath is a composite binary classifier trained separately for each diagnostic category (e.g. pneumonia vs not pneumonia etc.). It consists of a CNN audio classifier that produces predictions for single recordings, followed by a logistic regression that aggregates the predictions of the recordings corresponding to the eight anatomical sites, to output a single value per patient. Intermediate outputs, such as the segment-wise predictions for a recording, are extracted for later interpretability analysis. Figure 2 depicts the pipeline of the DeepBreath binary classifier.

Preprocessing - Generating fixed-size inputs (only during training). The training phase requires fixed-size inputs for batch-wise processing. Here, 5-second audio frames are presented to the classification architecture. Shorter recordings are zero-padded. For recording. The resting respiratory rhythm for an adult is around two seconds inhalation and three seconds exhalation [2]. For a child, a full respiration cycle is shorter, and respiratory disease tends to reduce this further. Thus, a 5 second crop would generally ensure at least one full respiration cycle irrespective of the random starting position. At inference time, the model can process the entire recording, no matter the duration. There is thus no need for zero-padding.

Spectral transformation-preprocessing. Digital Lungs Auscultation (DLAs) are first converted to log-mel spectrograms using torchaudio. The spectrograms are generated by computing discrete Fourier transforms (DFT) over short overlapping windows. A Hann window length of 256 and a hop length of 64 ms were used. At a 4000 Hz sampling rate, this corresponds to a window duration of 64 ms, and a hop duration of 16 ms. This process is known as the Short Time Fourier Transform (STFT). With a hop duration of 16 ms, we get 62.5 frames per second (rounded up in practice). To get log-mel spectrograms, the obtained magnitude spectra for frequencies between 250 and 750 Hz are projected onto 32 mel-bands, and converted to logarithmic magnitudes. Again, a narrow frequency range was chosen to reduce the interference of background noises. The log-mel spectrogram of a 5-second clip has a shape of $32 \times 313$. Before being processed by the CNN model, the log-mel spectrograms are normalized with Batch Normalization [3]. Because for spectrograms, the vertical translational invariance property does not hold (unlike standard images, spectrograms are structured and contain different information in the different frequency bands), each frequency band is normalized independently of the others. During training, SpecAugment [4] is performed. This augmentation technique, that was initially developed for speech processing, masks out randomly selected frequency and time bands.

First computation tool: in the present exemple, the first computation tool is CNN architecture of 5 convolutional blocks We adapted an architecture from the PANN paper [1] codebase. The original Cnn14_DecisionLevelAtt model was designed for sound event detection.. Each convolutional block consists of 2 convolutional layers with a kernel size of $3 \times 3$. Batch normalization is applied between each convolutional layer to speed up and stabilize the training. After each convolutional layer and Batch normalization, we use ReLU nonlinearity [5]. We apply average pooling of size $2 \times 2$ after the first 4 convolutional blocks for down-sampling, as $2 \times 2$ average pooling has been shown to outperform $2 \times 2$ max pooling [26]. After the last convolutional layer, the frequency dimension is reduced with average pooling. Average and max pooling of size 3 and stride 1 are applied over the time dimension and summed to get smoothed feature maps. Then a fully connected layer (with in_features = 1024) followed by a ReLU nonlinearity is applied to each of the time-domain feature vectors (of size 1024, which corresponds to the number of channels of the last convolutional layer).

Let x = {x1, ... , xT } be the sequence of feature vectors obtained from the previous step. Here T is the number of segments, which depends on the duration of the recording (note that T is determined by the duration of the input and the receptive field of our CNN model). In order to get segment-level predictions, an attention block is applied to the feature vectors. This attention block outputs two values per feature vector, by applying two distinct fully connected layers to each of the feature vectors (implemented with Conv1d in PyTorch). The first fully connected layer is followed by a sigmoid activation function and outputs a value p(xi). The second fully connected layer is followed by a Tanh nonlinearity and outputs a weight v(xi). These weights are then normalized over all segments with a softmax function:

$$g(x_i) = \frac{exp(v(x_i))}{\sum_{j=1}^{T} exp(v(x_j))}, i = 1, ..., T.$$

The value g(xi) is called the attention value of the ith segment. It controls how much the prediction p(xi) should be attended in the clip-level prediction. The clip-level prediction is now obtained as follows:

$$p(x) = \sum_{i=1}^{T} g(x_i)p(x_i), thus: p(x) \in [0,1].$$

Dropout [6] is applied after each downsampling operation and fully connected layers to prevent the model from overfitting.

Second computation tool - logistic regression: To obtain patient-level predictions, we combine the predictions of each of the 8 anatomic sites of a single patient and concatenate them to obtain a vector of size 8. In this step, only patients for which all eight recordings are available were selected. This new set of features can be used to construct new datasets for the train, tune and test folds. We chose to fit a logistic regression model to these new features, because it has properties pertinent to our setting, such as being interpretable and returning the probability of an event occurring. With this model, there is no need for a tune set to save the best model during training. Thus we concatenated the feature matrices of the train and tune folds, to obtain a new feature matrix that will be used to fit the logistic regression model. The test features will be used to evaluate our model. Note that this is the first time that the test data is used. The CNN model is not trained during this second phase and never sees the test data.

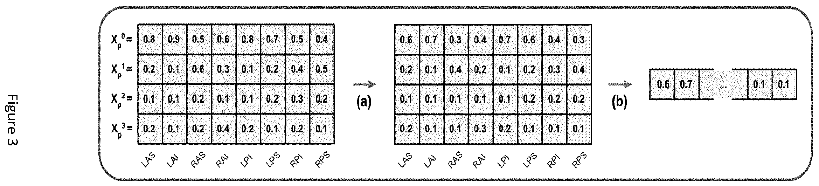

For diagnostic classification, we combine the positional feature vectors (corresponding to the eight anatomical sites) of the four CNN audio classifiers. The feature vectors are concatenated to form a prediction array of size 4 × 8, and the columns are then L1-normalized (for each column, the sum of its values is equal to 1). The idea behind this normalization is that recordings-which were identified by multiple CNN models as having a high likelihood of belonging to their respective class-should matter less in the aggregated patient prediction. Similar to the aggregation step in the binary models, we obtain new feature datasets for the training, tune and test folds. Figure 3 shows how that data is generated. For every patient, the four binary models produce a feature vector of size 8, corresponding to the predictions of the recordings from the 8 anatomical sites. Those feature vectors are concatenated to form a prediction array of size 4 (classes) × 8 (sites). Then, the following operations are applied to the prediction array: (a) Column normalization of the prediction array (b) Flattening to obtain a feature vector of size 32. The final feature vector is than given as input to the multinomial logistic regression. A multinomial logistic regression is then trained to predict a patient's diagnosis. Again, the test features are only used to evaluate the model The final feature vector is than given as input to the multinomial logistic regression.

Model training / Optimization: The CNN classifiers were trained with a Binary Cross Entropy (BCE) loss with clip-level predictions and labels. A batch-size of 64, and an AdamW optimizer [7], combined with a 1cycle learning rate policy [8], were used. The 1cycle policy increases the learning rate (LR) from an initial value to some maximum LR and then decreases it from that maximum to some minimum value, much lower than the initial LR. The maximum LR was set to 0.001. The default parameters of the PyTorch implementation were used otherwise. The weight decay coefficient of the AdamW optimizer was set to 0.005.

Class imbalance. A balancing sampling strategy was implemented to address the class imbalance, w.r.t. the diagnoses and recording centers. First, when training the model to identify one of the four categories, it is important that the three other categories are equally represented. This is because otherwise, the model might learn to distinguish between two categories, which is easier. Second, sampling recordings from different locations in a balanced way is also important. Our experiments have shown that the model can learn to distinguish recording locations with relatively high accuracy. Thus, there is a risk that the model may learn spurious features when training it to distinguish pathologies by focusing on center-specific background noise for over-represented categories in one location. Hence, our sampling strategy aims to enforce that the CNN learns location-invariant representations, as much as possible, as some localized features might yet be desired. With a balanced sampling, an epoch longer corresponds to a full sweep of the data. The number of batches per epoch is set such that N recordings are sampled during an epoch, where N is the total number of recordings. With over-sampling (or under-sampling), we are bound to have duplicates over an epoch (or missed recordings, respectively).

Evaluation Every model was trained for 100 epochs. After 60 epochs, the model performance is evaluated on the tune fold, to save the best model w.r.t. mean positional AUROC, obtained as follows. For every recording in the tune fold, the CNN classifier predicts a score in [0, 1]. Those scores are then split into eight different groups, depending on the position that was used for the recording. For every position, AUROC is computed on the positional scores, and then the mean of these eight AUROC values is returned. The idea behind this metric is that the model might perform differently depending on the position (more confident for certain positions than for others), and since we are feeding the positional scores to another model (logistic regression), metrics that require us to binarize the scores like accuracy or F1-score might not be adapted to the task.

Inference Optimization. As DeepBreath can perform inference on variable lengths and anatomical positions of audio, we determine the minimum length and smallest combination of recordings that the trained DeepBreath model requires to ensure its reported performance. These experiments are performed exclusively on the test set.

Minimal position combination. We first select the best combination of anatomical positions from the 8 available positions using recursive feature elimination. For each number of positions (between 2 and 7), the most frequently selected combination across 20 nested cross-validation iterations were retained as the best. This is compared to a random selection. Each combination is performed within a specific patient and for each disease-specific model.

Minimal inference duration. To then explore the minimum duration required for each combination (and the randomised control), we create variable duration crops, ranging between 1 and 30 seconds in 2.5 second increments.

Statistical methods. For every cross validation iteration, sensitivity, specificity and area under the receiver-operator-characteristic curve (AUROC) values are computed on the internal test fold and the external validation data. Class recalls (w.r.t. the actual diagnosis, instead of considering only binary labels) are also computed. With nested cross-validation, this procedure is repeated 20 times. CI95% are reported for all metrics.

When combining the outputs of the trained CNN classifiers for diagnostic classification, confusion matrices were used to visualize the classification performance. For every combination of train, tune and test folds, we get a confusion matrix for both the internal test fold and the external validation data. Those confusion matrices are then summed, resulting in two final confusion matrices. Confusion matrices are row-normalized to highlight class performance.

Results

DeepBreath -Binary predictions : The four binary submodels of DeepBreath were evaluated on both internal test folds and external validation data. The results are reported in Table 2 below. The model that discriminates healthy from pathological patients achieved an internal test AUROC, sensitivity and specificity of 0.930, 0.849 and 0.840 respectively. On the external validation, the model lost only between 7 and 8% for each metric. Significant differences in performance were seen among disease classes. Pneumonia showed the lowest performance, with an internal test AUROC of 0.754 (0.521 sensitivity and 0.852 specificity). External validation data, presented similar performance with losses of less than 6% for each metric. The model designed for wheezing disorders was much more performant with an AUROC of 0.915, sensitivity of 0.805 and specificity of 0.884. However, this model lost much more performance in external validation, with a nearly 20% drop for AUROC and sensitivity. Finally, the bronchiolitis submodel had an internal test AUROC of 0.938, sensitivity of 0.820 and specificity of 0.886. On external validation, the model had a similar AUROC with a loss of only 7.3% but with a redistribution of sensitivity and specificity at 0.488 (a loss of 33.2%) and 0.934 (a gain of 4.8%). The specificity vs sensitivity trade-off could be specified a priori, but was not performed in this work.

Table 2

| Target | Validation | Sensitivity (CI95) | Specificity (CI95) | AUROC (CI95) |
|---|---|---|---|---|
| Control | Internal | 0.849 (0.825-0.873) | 0.840 (0.813-0.868) | 0.930 (0.922-0.937) |
| | External | 0.780 (0.720-0.839) | 0.763 (0.716-0.810) | 0.859 (0.844-0.873) |
| Pneumonia | Internal | 0.521 (0.428-0.614) | 0.852 (0.831-0.873) | 0.754 (0.708-0.800) |
| | External | 0.471 (0.436-0.507) | 0.809 (0.788-0.830) | 0.695 (0.686-0.704) |
| Wheezing Disorder | Internal | 0.805 (0.769-0.841) | 0.884 (0.867-0.901) | 0.915 (0.899-0.930) |
| | External | 0.608 (0.567-0.649) | 0.696 (0.658-0.734) | 0.725 (0.712-0.739) |
| Bronchiolitis | Internal | 0.820 (0.784-0.856) | 0.886 (0.863-0.909) | 0.938 (0.928-0.948) |
| | External | 0.488 (0.455-0.520) | 0.934 (0.927-0.941) | 0.865 (0.860-0.871) |

DeepBreath returned promising results, discriminating several disease classes with over 90% area under the receiver operating characteristic (AUROC) curve, and losing less than 10% on external validation. Even the binary classifier for pneumonia achieved over 75% AUROC with only 5% loss in external validation. This value is considered surprisingly good in light of the notorious lack of international gold standard for the diagnosis of the disease and the international context of our cohort. DeepBreath also showed potential to maintain stable performance under sparse data conditions. Indeed, using just 5-second recordings from any 4 anatomic positions on the thorax was equivalent to using 30 second clips from all 8 positions.

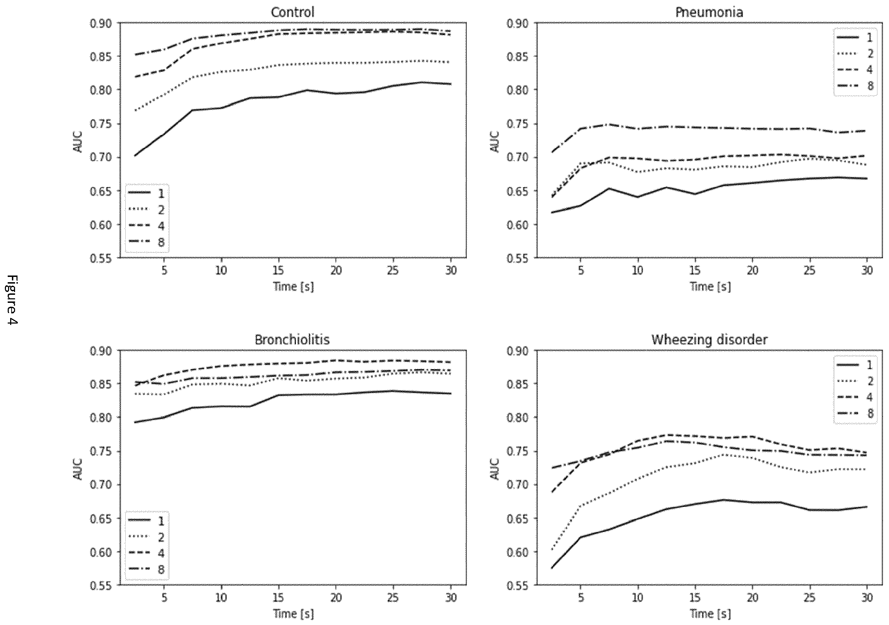

Figure 4 shows the performance of the binary DeepBreath models on variable lengths and anatomical combinations of auscultation audio recordings. Only samples with at least 30 seconds and 8 positions available are used, resulting in a slight deviation from the overall results. For each binary model, we explore the minimal number of anatomical positions (from 1 to 8) in its most informative combination using recursive feature elimination (RFE, depicted as solid lines). Each sample is then cropped to various durations (from 1 second to 30 seconds) and the AUROC of each combination and duration is plotted. We see that performance significantly decreases when using only 1-3 positions compared with the baseline of all 8 positions. However, 4 or more positions do not have a significantly different performance from this baseline. Further, the AUROC is maintained for all samples until about 3-5 seconds. Testing a random combination of 4 positions was also not significantly different from the RFE-selected position combination. Thus maintains performance when given at least 5 seconds of 4 randomly selected positions.

The interpretable-by-design approach of attention-spectogram mapping further validates the clinical relevance of our results, offering unique insights into the model's decision-making process. These tangible visualizations map the predictive attention on the audio spectrogram allowing it to be aligned with human annotations of the respiration cycle. We found intuitive concordance with inspirations and expirations, which better ensures that discrimination is based on respiratory signals rather than spurious noise. Thus, DeepBreath can be interrogated by medical experts, allowing them to make an informed assessment of the plausibility of the model's output. This interpretation technique could possibly be used as a more objective way to identify adventitious sounds, requiring no (costly) labeling of short audio segments, but using only clip-level annotations. Extrapolating the possible applications of this approach, it could even provide a method for standardising the unique acoustic characteristics of respiratory disease into a visually interpretable set of patterns that could find a use in medical training.

While the embodiments have been described in conjunction with a number of embodiments, it is evident that many alternatives, modifications and variations would be or are apparent to those of ordinary skill in the applicable arts. Accordingly, this disclosure is intended to embrace all such alternatives, modifications, equivalents and variations that are within the scope of this disclosure. This for example particularly the case regarding the different apparatuses which can be used.

References:

1. Kong Q, Cao Y, Iqbal T, Wang Y, Wang W, Plumbley MD. PANNs: Large-scale pretrained audio neural networks for audio pattern recognition. IEEE/ACM Transactions on Audio, Speech, and Language Processing. 2020;28:2880-2894.

2. Barret KE, Boitano S, Barman SM. Ganong's review of medical physiology. McGraw-Hill Medical; 2012.

3. Ioffe S, Szegedy C. Batch normalization: Accelerating deep network training by reducing internal covariate shift. In: International conference on machine learning. PMLR; 2015. p. 448-456.

4. Park DS, Chan W, Zhang Y, Chiu CC, Zoph B, Cubuk ED, et al. Specaugment: Asimple data augmentation method for automatic speech recognition. arXiv preprint arXiv: 190408779. 2019;.

5. Nair V, Hinton GE. Rectified linear units improve restricted boltzmann machines. In: Icml; 2010.

6. Srivastava N, Hinton G, Krizhevsky A, Sutskever I, Salakhutdinov R. Dropout: a simple way to prevent neural networks from overfitting. The journal of machine learning research. 2014; 15(1): 1929-1958.

7. Loshchilov I, Hutter F. Decoupled weight decay regularization. arXiv preprint arXiv:171105101. 2017;.

8. Smith LN, Topin N. Super-convergence: Very fast training of neural networks using large learning rates. In: Artificial Intelligence and Machine Learning for Multi-Domain Operations Applications. vol. 11006. International Society for Optics and Photonics; 2019. p. 1100612.

**Claims**

1. Computer implemented method for detecting a disease in a patient, the method comprising

providing chest recordings recorded during chest auscultation of the patient,

processing the chest recordings with a computational tool configured for recognizing audible signature corresponding to the disease,

detecting the presence or the absence of said disease depending on the output of the computational tool,

the method being **characterized in that**

the chest recordings are recorded from a plurality of distinct acquisition sites distributed on the patient's chest, each site corresponding to one recording;

and **in that** the processing of the chest recordings further comprises

> i) applying a first computation tool on the chest recordings of each of acquisition site to compute a first score for each recording of each of acquisition site;
> ii) aggregating all the first scores computed for the patient by applying a second computational tool to compute a global score for the patient for said disease;
> iii) determining the presence or the absence of the disease based on the global score.

2. Method according to claim 1, wherein the first computation tool comprises at least one computation model trained for a specific disease and one model trained for identifying healthy patient so that the first computation tool provides a first score for each computation model for each site to compute the global score for the specific disease for the patient.

3. Method according to claim 2, wherein the first computation tool comprises several computation models each trained for a specific disease and one model trained for identifying healthy patient so that the first computation tool provides a first score for each computation model for each disease for each acquisition sites, to compute the global score for each specific disease for the patient.

4. Method according to claims 2 or 3, wherein the computation model of the first computation tool comprises at least an audio classifier, preferably an CNN architecture comprising a plurality of convolutional blocks.

5. Method according to any one of claims 1 to 4, wherein the processing of the chest recordings further comprise a pre processing step comprising

> a high pass filter to filter sounds of the chest recording below a cut-off frequency preferably of about 100 Hz, more preferably of about 125 Hz, more preferably of about 150 Hz,
> and/or, preferably and
> a low pass filter to filter sounds of the chest recording above a cut-off frequency preferably of about 1000 Hz, more preferably of about 900 Hz, more preferably of about 800 Hz.

6. Method according to any one of claims 1 to 5, where the processing of the chest recordings further comprise a pre processing step comprising

filtering the chest recording to select the sound(s) relevant for the disease to be detected.

7. Method according to any one of claims 1 to 6, wherein each chest recording comprises an audio signal, the processing of the chest recordings further comprises

converting each audio signal into a two dimensions image preferably a spectrogram, preferably a log-mel spectrogram for instance by applying a Discrete Fourier transform on each audio signal.

8. Method according to any one of claims 1 to 7, wherein the computation of the first score for each acquisition site comprises

> calculating segment-wise outputs for each site
> aggregating the segment-wise outputs for each site into a single clip wise output as first score for each acquisition site
> using said clip wise first score as input for the second computation tool.

9. Method according to any one of claims 1 to 8, wherein the computation model of the second computation tool comprises a logistic regression configured for aggregating all the first score of each acquisition site into a global score for the patient.

10. Method according to any one of claims 1 to 9, wherein the determination step comprises

attributing a threshold score for said disease and
comparing the global score of the patient to the threshold score for said disease and
determining that the patient is positive for said disease if the global score of the patient is above the threshold score and that the patient is negative for said disease if the global score of the patient is below the threshold score.

11. Method according to any one of claims 1 to 10, wherein the plurality of distinct acquisition sites is chosen among the list comprising sites in the right chest, sites in the left chests, in the superior part of the chests, sites in the inferior part of the chests, site in the anterior part of the chest, site in the posterior part of the chest.

12. Method according to any one of claims 1 to 11, wherein the plurality of distinct acquisition sites comprises at least 4 acquisitions sites, preferably between about 2 and 20 sites, preferably between 2 to 10, more preferably 4, more preferably 8.

13. Method according to any one of claims 1 to 12, wherein the duration of each recording is at least 3 seconds, preferably between about 3 seconds and 1 minute, preferably at least about 5 seconds, preferably between about 5 seconds and 35 seconds.

14. A computer program comprising instructions which, when the program is executed by a computer, cause the computer to carry out the method according to any one of claims 1 to 13.

15. An apparatus comprising means for carrying out the method of according to any one of claims 1 to 13.

Figure 1

Figure 2

Figure 3

Figure 4

Europäisches
Patentamt

European
Patent Office

Office européen
des brevets

# EUROPEAN SEARCH REPORT

Application Number

EP 22 20 5202

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2012/132211 A1 (HALPERIN AVNER [IL] ET AL) 31 May 2012 (2012-05-31) <br> * paragraphs [0611], [0612], [0739] – [0741] * <br> * figures 4A-4C * <br> ----- | 1-15 | INV. <br> A61B7/00 <br> A61B5/08 |

TECHNICAL FIELDS
SEARCHED     (IPC)

A61B

The present search report has been drawn up for all claims

| Place of search | Date of completion of the search | Examiner |
|---|---|---|
| Munich | 31 March 2023 | Hemb, Björn |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

.......................................................................

& : member of the same patent family, corresponding
    document

EPO FORM 1503 03.82 (P04C01)

EP 4 364 669 A1

## ANNEX TO THE EUROPEAN SEARCH REPORT
## ON EUROPEAN PATENT APPLICATION NO.

EP 22 20 5202

This annex lists the patent family members relating to the patent documents cited in the above-mentioned European search report.
The members are as contained in the European Patent Office EDP file on
The European Patent Office is in no way liable for these particulars which are merely given for the purpose of information.

31-03-2023

| Patent document cited in search report | Publication date | Patent family member(s) | Publication date |
|---|---|---|---|
| US 2012132211 A1 | 31-05-2012 | EP 2142095 A1 | 13-01-2010 |
| | | US 2008275349 A1 | 06-11-2008 |
| | | US 2012132211 A1 | 31-05-2012 |
| | | WO 2008135985 A1 | 13-11-2008 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **KONG Q ; CAO Y ; IQBAL T ; WANG Y ; WANG W ; PLUMBLEY MD.** PANNs: Large-scale pretrained audio neural networks for audio pattern recognition. *IEEE/ACM Transactions on Audio, Speech, and Language Processing.*, 2020, vol. 28, 2880-2894
- **BARRET KE ; BOITANO S ; BARMAN SM.** Ganong's review of medical physiology. McGraw-Hill Medical, 2012
- **LOFFE S ; SZEGEDY C.** Batch normalization: Accelerating deep network training by reducing internal covariate shift. *International conference on machine learning. PMLR,* 2015, 448-456
- **PARK DS ; CHAN W ; ZHANG Y ; CHIU CC ; ZOPH B ; CUBUK ED et al.** Specaugment: Asimple data augmentation method for automatic speech recognition. *arXiv: 190408779,* 2019
- **NAIR V ; HINTON GE.** Rectified linear units improve restricted boltzmann machines. *Icml,* 2010
- **SRIVASTAVA N ; HINTON G ; KRIZHEVSKY A ; SUTSKEVER I ; SALAKHUTDINOV R.** Dropout: a simple way to prevent neural networks from overfitting. *The journal of machine learning research.,* 2014, vol. 15 (1), 1929-1958
- **LOSHCHILOV I ; HUTTER F.** Decoupled weight decay regularization. *arXiv:171105101,* 2017
- Super-convergence: Very fast training of neural networks using large learning rates. **SMITH LN ; TOPIN N.** Artificial Intelligence and Machine Learning for Multi-Domain Operations Applications. International Society for Optics and Photonics, 2019, vol. 11006, 1100612